# EUROPEAN PATENT APPLICATION

(11) **EP 1 584 691 A1**
(43) Date of publication of application: **12.10.2005**
(21) Application number: 03785985.7
(22) Date of filing: 29.12.2003
(51) Int. Cl.: C12Q 1/68

(54) **DEVICE FOR ASSAYING POLYMORPHISMS OF GENE XPD/ERCC2 FOR THE CORRECT ADMINISTRATION OF CHEMOTHERAPY FOR LUNG CANCER**

(30) Priority: 10.01.2003 ES 200300054
(71) Applicant: Fundacion para la Investigacion Clinica y Molecular del Cancer de Pulmon, 08036 Barcelona (ES)
(72) Inventor: ROSELL COSTA, Rafael, 08036 Barcelona (ES); TARON ROCA, Miguel, 08036 Barcelona (ES)
(74) Representative: Elzaburu Marquez, Alberto
(86) International application number: PCT/ES2003/000666
(87) International publication number: WO 2004/063395

(57) **Abstract**

The invention is encompassed in the technical sector of lung cancer treatment with antitumor drugs, and it specifically develops a diagnostic device which allows treating each patient with the most effective drug according to the polymorphism they show for the XPD gene. The assay device of the invention is based on the polymorphic variants of the XPD gene at exon 23 (A-C, Lys 751 Gln) and at exon 10 (G-A, Asp312Asn) and on the development of specific primers which allow detecting said polymorphisms by PCR or by means of automatic DNA sequencing.

LIST OF SEQUENCES

<110> FUNDACIÓN PARA LA INVESTIGACIÓN CLINICA Y MOLECULAR
DEL CANCER DE PULMÓN (LUNG CANCER CLINICAL AND MOLECULAR
RESEARCH FOUNDATION)
<120> "ASSAY DEVICE OF XPD/ERCC2 GENE POLYMORPHISMS FOR THE
CORRECT ADMINISTRATION OF CHEMOTHERAPY IN LUNG CANCER"
<130> PCT-152
<160> 8
<210> SEQ ID NO.:1
<211> 15
<212> oligonucleotide
<213> artificial sequence
<220>
<221> primer
<400>
acgcccacct ggcca                                15

<210> SEQ ID NO.: 2
<211> 17
<212> oligonucleotide
<213> artificial sequence
<220>
<221> primer
<400>
ggcgggaaag ggactgg                              17

<210> SEQ ID NO.: 3
<211> 19
<212> oligonucleotide
<213> artificial sequence
<220>
<221> primer
<223> TaqMan MGB™ VIC Probe
<400>

ccgtgctgcc cgacgaagt                                                19

<210> SEQ ID NO.: 4
<211> 19
<212> oligonucleotide
<213> artificial sequence
<220>
<221> primer
<223> TaqMan MGB™ 6-FAM Probe
<400>
cccgtgctgc ccaacgaag                                                19

<210> SEQ ID NO.: 5
<211> 22
<212> oligonucleotide
<213> artificial sequence
<220>
<221> primer
<400>
gcctggagca gctagaatca ga                                            22

<210> SEQ ID NO.: 6
<211> 22
<212> oligonucleotide
<213> artificial sequence
<220>
<221> primer
<400>
cactcagagc tgctgagcaa tc                                            22

<210> SEQ ID NO.: 7
<211> 16
<212> oligonucleotide
<213> artificial sequence
<220>

<221> primer
<223> TaqMan MGB™ VIC Probe

<400>
tatcctctgc agcgtc                                16
<210> SEQ ID NO.: 8
<211> 17
<212> oligonucleotide
<213> artificial sequence
<220>
<221> primer
<223> TaqMan MGB™ 6-FAM Probe
<400>
ctatcctctt cagcgtc                               17

## Description

### Scope of the Invention

The invention is encompassed within the technical field of lung cancer treatment with antitumor drugs and, specifically, develops a diagnostic device which allows for treating each patient with the most effective drug according to the polymorphism they show for the XPD gene.

### State of the Art

Different antitumor drugs damage DNA in a manner similar to that carried out by carcinogens. The covalent bond of the carcinogen or of a cytotoxic antitumor drug provides the formation of a DNA base which is chemically altered, which is known with the term adduct (Philips, 2002). Cisplatin causes bonds between DNA strands, and such adducts provide the cytotoxic action of cisplatin (Siddik, 2002). DNA repair systems are essential for eliminating cisplatin adducts. Nucleotide Excision Repair (NER) is the main pathway for protecting the host from developing lung cancer, and at the same time it is the generating principle of resistance to cisplatin. In fact, both the benzopyrene diol epoxide (BPDE) adducts and also the cisplatin adducts effectively block RNA polymerase II and thus void transcription (Hanawalt, 2001). These DNA lesions are eliminated by the NER system, which in turn is subdivided into two metabolic pathways: Transcription Coupled Repair (TCR) and Global Genomic Repair (GGR) (Diagram 1). TCR (or TC-NER) significantly repairs the lesions blocking transcription in the strand transcribing the DNA of active genes, whereas GGR (or GG-NER) repairs the lesions in the strand which does not transcribe in the active genes and also in the genome without transcription function (Cullinane et al., 1999; May et al., 1993; McKay et al., 1998).

### Diagram 1 : Representation of the nucleotide excision repair (NER) pathways.

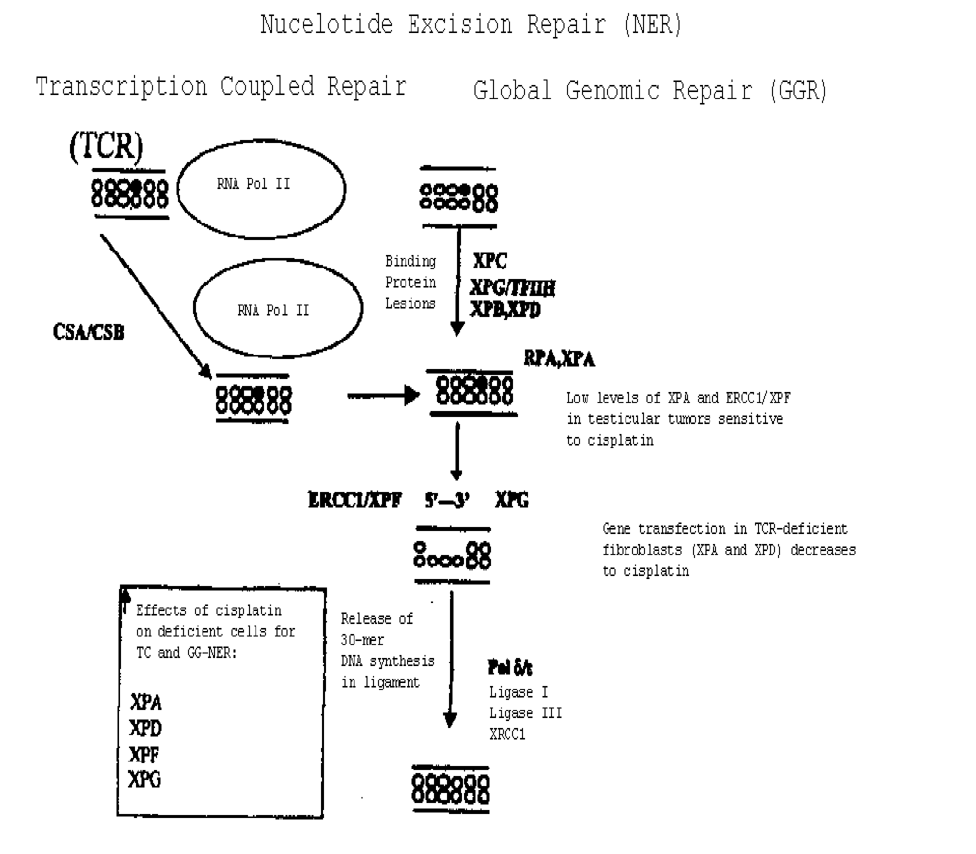

In human beings, NER is a fundamental defense mechanism against the carcinogenic effects of sunlight, and certain genetic defects in the repair pathways produce severe consequences on autosomal recessive hereditary disorders, such as xeroderma pigmentosum (XP) . In fact, patients with this disease are hypersensitive to sunlight with an extraordinary susceptibility to and high frequency of suffering from skin cancer. In XP, there are seven complementary groups which can be deficient in the NER pathways. These genes are enumerated from XPA to XPG. In XP disease, these genes are defective in both NER pathways (Conforti et al., 2000). In ovarian cancer and, less frequently, in colon cancer and lung cancer, losses of heterozygosity have been observed in different XP genes (Takebayashi et al., 2001). The loss of heterozygosity is related to the loss of transcription, and the deficiency of these genes entails an increase in sensitivity to cisplatin, as has been observed in ovarian cancer. Cockayne Syndrome (CS) is another photosensitive disease which is linked to a deficiency in the NER system. Two genes have been identified, CSA and CSB. The alterations of said genes disrupt the functions in which they are involved in the TCR pathway (Conforti et al., 2000).

The left portion of Diagram 1 (modified from Rajewsky and Müller, 2002) shows the TCR pathway which is the essential pathway for detecting the damage caused by cisplatin (Cullinane et al., 1999). In the moment of transcription, when the RNA polymerase II detects the lesion, the specific CSA and CSB transcription factors are activated in the molecular NER pathway (Furuta et al., 2002; McKay et al., 2001). The XP genes are also involved in the TCR pathway, as shown in the box in Diagram 1. Essentially, different molecular deficiencies in both pathways (GGR and TCR) in fibroblasts confer an increase in the sensitivity to the cytotoxic effect of cisplatin in comparison to what occurs in normal fibroblasts. What is important is that any deficiency in any of the XPA, XPD, XPF or XPG genes confers a substantial increase of the activity of cisplatin (Furuta et al., 2002).

As a common principle, the repertoire of cytotoxins used in cancer treatment, particularly in lung cancer, are centered around the use of cisplatin or carboplatin in association with another drug, such as gemcitabine, docetaxel, paclitaxel or vinorelbine as the most important ones and of standard clinical use. However, chemotherapy results in metastatic lung cancer are very limited, with a median time to progression which does not pass five months, and a median survival which does not exceed eight or ten months. No type of combination stands out in improving such survival expectancies. However, on an individual level, as a clinical verification, it is noted that individual cases have significantly longer survivals. Polymorphisms, which are simple nucleotide changes, confer interindividual differences which alter gene expression or function. Such polymorphisms existing in a very high proportion in the genome are still under study. It is possible that more than 3,000 polymorphisms will be characterized in the future which will be useful for determining susceptibility to cancer, the prognostic value of the disease and the predictive value of response to treatment. At the level of messenger RNA expression, it has been verified that the overexpression of the ERCC1 gene acting in the GGR pathway causes resistance to cisplatin in gastric, ovarian and lung cancer (Lord et al., 2002; Metzger et al., 1998; Shirota et al., 2001).

XPD polymorphisms have been linked to a decrease in DNA repair capacity in different studies (Spitz et al., 2001). In fact, about half the population has the Lys751Lys genotype, and they also have the normal, homozygote Asp312Asp genotype. Such patients or persons with normal homozygote genotype have a very good repair capacity and, therefore, can be resistant to cisplatin (Bosken et al., 2002). The increase of the repair capacity, which can be measured by means of functional assays, has been associated with the resistance to cisplatin in non small cell lung cancer (NSCLC) (Zeng-Rong et al., 1995). Repair capacity has also been studied by means of measuring the reactivation of a gene damaged by exposure to BPDE, and repair capacity levels are significantly lower in lung cancer patients than in control patients (Wei et al., 1996, 2000). Multiple studies indicate that the decline of the repair capacity and the increase in the DNA adduct levels increases the risk of lung cancer. Therefore, the basal expression of critical genes in the NER pathway is related to the risk of lung cancer. By RT-PCR, the ERCC1, XPB, XPG, CSB and XPC transcript levels were measured in lymphocytes of 75 lung cancer patients and 95 control patients. The results showed a significant decrease in the XPG and CSB expression levels in the cases of lung cancer in comparison with the controls (Cheng et al., 2000). What is very important is that the lymphocyte messenger RNA levels of the XPA, XPB, XPC, XPD, XPF, XPG, ERCC1 and CSB genes showed a very significant correlation in the messenger RNA levels between ERCC1 and XPD, in turn, the expression of both genes is correlated to DNA repair capacity (Vogel et al., 2000).

There are patents (WO 97/25442) relating to lung cancer diagnosis methods, as well as to diagnosis methods for other types of tumors (WO 97/38125, WO 95/16739) based on the detection of other polymorphisms different from those herein described. Other patents have also been located which also use the detection of polymorphisms in other genes to know the response of certain patients to other drugs (statins); but this applicant is not aware of patents determining which patients with lung cancer are more prone to one antitumor treatment or another.

### Literature

1. Aloyz R, Xu ZY, Bello V, et al. Regulation of cisplatin resistance and homologous recombinational repair by the TFIIH subunit XPD. Cancer Res 2002; 62:5457-5462
2. Bosken CH, Wei Q, Amos CI, Spitz MR:An analysis of DNA repair as a determinant of survival in patients with non-small-cell lung cancer. J Natl Cancer Inst 2002; 94:1091-1099
3. Cheng L, Guan Y, Li L, et al. Expression in normal human tissues of five nucleotide excision repair genes measured simultaneously by multiplex reverse transcription-polymerase chain reaction. Cancer ,epidemiology biomarkers & prevention 1999; 8:801-807
4. Cheng L, Guan Y, Li L, et al. Expression in normal human tissues of five nucleotide excision repair genes measured simultaneously by multiplex reverse transcription-polymerase chain reaction. Cancer, Epidemiol Biomark Prcv 8:801-807, 1999
5. Cheng L, Sptiz MR, K Hong W, Wei K. Reduced expression levels of nucleotide excision repair genes in lung cancer: a case-control analysis. Carcinogenesis 2000; 21: 1527-1530
6. Cheng L, Sptiz MR, K Hong W, Wei K. Reduced expression levels of nucleotide excision repair genes in lung cancer: a case-control analysis. Carcinogenesis 21: 1527-1530, 2000
7. Cheng L, Sturgis EM, Eicher SA, Sptiz MR, Wei Q. Expression of nucleotide excision repair genes and the risk for squamous cell carcinoma of the head and neck. Cancer 2002; 94:393-397
8. Conforti G, Nardo T, D'Incalci M, Stefanini M: Proneness to UV-induced apoptosis in human fibroblasts defective in transcription coupled repair is associated with the lack of Mdm2 transactivation. Oncogene 2000; 19:2714-2720
9. Cullinane C, Mazur SJ, Essigmann JM, et al: Inhibition of RNA polymerase II transcription in human cell extracts by cisplatin DNA damage. Biochemistry 1999; 38: 6204-6212
*10.* Furuta T, Ueda T, Aune G, et al. Transcription-coupled nucleotide excision repair as a determinant of cisplatin sensitivity of human cells. Cancer Res. 62:4809-4902, 2002
11. Furuta T, Ueda T, Aune G, et al: Transcription-coupled nucleotide excision repair as a determinant of cisplatin sensitivity of human cells. Cancer Res 2002; 62:4899-4902
12. Hanawalt PC: Controlling the efficiency of excision repair.Mut Res 2001;485:3-13
13. Hou S-M, Fält S, Angelini S, et al: The XPD variant alleles are associated with increased aromatic DNA adduct level and lung cancer risk. Carcinogenesis 2002;23:599-603
14. Lord RVN, Brabender J, Gandara D, et al: Low ERCC1 expression correlates with prolonged survival after cisplatin plus gemcitabine chemotherapy in non-small-cell lung cancer. Clin Cancer Res 2002; 8: 2286-2291
15. May A, Naim RS, Okumoto DS, et al: Repair of individual DNA strands in the hamster dihydrofolate reductase gene after treatment with ultraviolet light, alkylating agents, and cisplatin. J Biol Chem 1993;268-1650-1657
16. McKay BC, Becerril C, Ljungman M: p53 plays a protective role against UV-and cisplatin-induced apoptosis in transcription-coupled repair proficient fibroblasts. Oncogene 2001; 20:6805-6808
17. McKay BC, Ljungman M, Rainbow AJ: Persistent DNA damage induced by ultraviolet light inhibits p21^{waFL} and bax expression: implications for DNA repair, UV sensitivity and the induction of apoptosis. Oncogene 1998 ;17:545-555
18. Metzger R, Leichman CG, Danenberg KD, et al: ERCC1 mRNA levels complement thymidylate synthase mRNA levels in predicting response and survival for gastric cancer patients receiving combination cisplatin and fluorouracil chemotherapy. J Clin Oncol 1998; 16:309-316
19. Phillips DH: The formation of DNA adducts. In: Alison MR, ed. The Cancer Handbook. London: Nature Publishing Group; 2002:293-307
20. Rajewsky MF, Müller R. DNA repair and the cell cycle as targets in cancer therapy. In: Alison MR, ed. The Cancer Handbook. London: Nature Publishing Group 2002; 1507-1519
21. Shirota Y, Stoehlmacher J, Brabender J, et al: ERCC1 and thymidylate synthase mRNA levels predict survival for colorectal cancer patients receiving combination oxaliplatin and fluorouracil chemotherapy. J Clin Oncol 2001; 19:4298-4304
22. Siddik ZH: Mechanisms of action of cancer chemotherapeutic agents: DNA-interactive alkylating agents and antitumour platinum-based drugs. In: Alison MR, ed. The Cancer Handbook. London: Nature Publishing Group; 2002:1295.1313
*23.* Spitz MR, Wu X, Wang Y, et al. Modulation of nucleotide excision repair capacity by XPD polymorphisms in lung cancer patients. Cancer Res 61:1354-1357, 2001
24. Spitz MR, Wu X, Wang Y, et al: Modulation of nucleotide excision repair capacity by XPD polymorphisms in lung cancer patients. Cancer Res 2001; 61:1354-1357
25. Takebayashi Y, Nakayama K, Kanzaki A, et al: Loss of heterozygosity of nucleotide excision repair factors in sporadic ovarian, colon and lung carcinomas: implication for their roles of carcinogenesis in human solid tumors. Cancer Letters 2001; 174:115-125
26. Vogel U, Dybdahl M, Frentz G, et al. DNA repair capacity: inconsistency between effect of over-expression of five NER genes and the correlation to mRNA levels in primary lymphocytes. Mutat Res. 461.197-210, 2000
27. Wei Q, Cheng L, Amos CI, et al. Repair of tobacco carcinogen-induced DNA adducts and lung cancer risk: a molecular epidemiologic study. J Natl Cancer Inst 2000; 92: 1764-1772
28. Wei Q, Cheng L, Ki Hong W, Spitz MR. Reduced DNA repair capacity in lung cancer patients. Cancer Res 1996; 56:4103-4107
29. Zeng -Rong N, Paterson J, Alpert P, et al. Elevated DNA capacity is associated with intrinsic resistance of lung cancer to chemotherapy. Cancer Res 1995; 55:4760-4764.

### Brief Description of the Invention

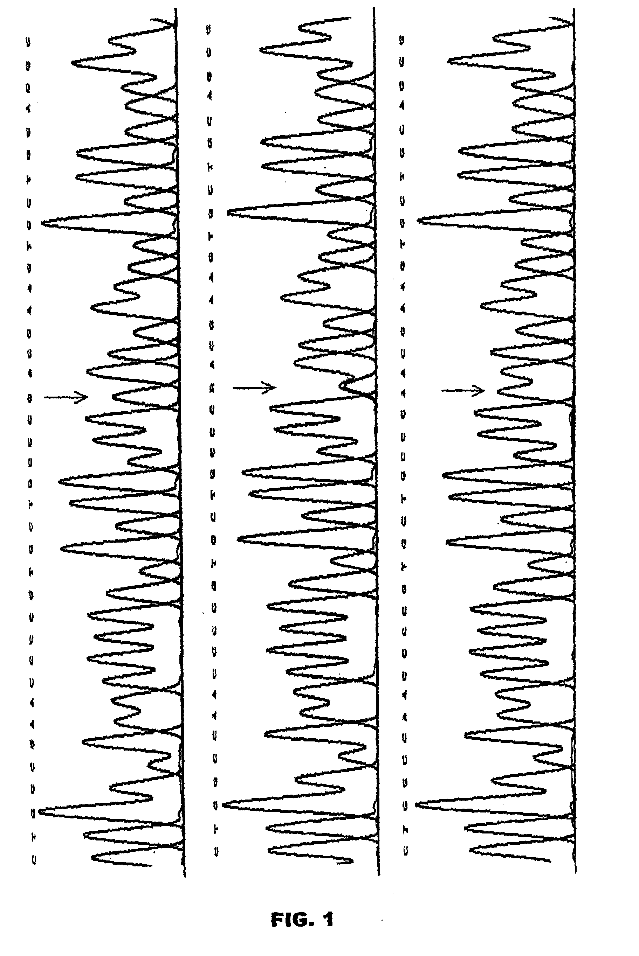

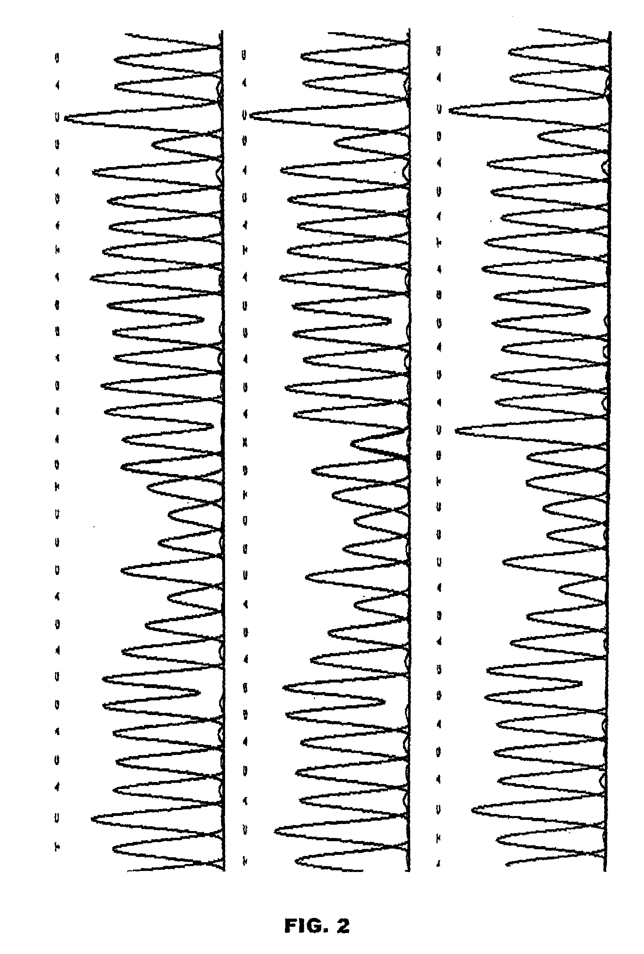

In the research carried out, the pharmacogenetic predictive value of XP gene polymorphic variants have been discovered. The XPD gene polymorphisms at exon 23 (A-C, Lys751Gln) and at exon 10 (G-A, Asp312Ans) have been studied. Figures 1 and 2 show two examples of identification of the XPD polymorphisms at condons 312 and 751, respectively, carried out by automatic sequencing. Diagram 2 shows the different DNA repair metabolic pathways and the position occupied by the XPD gene in said pathways. The clinical interest in examining XPD polymorphism is strengthened, given that a screening of a panel of cell lines of different tumors of the National Cancer Institute reveals that among XPA, XPB, XPD and ERCC1, only the overexpression of XPD is correlated with resistance to alkylating agents (Aloyz et al., 2002) .

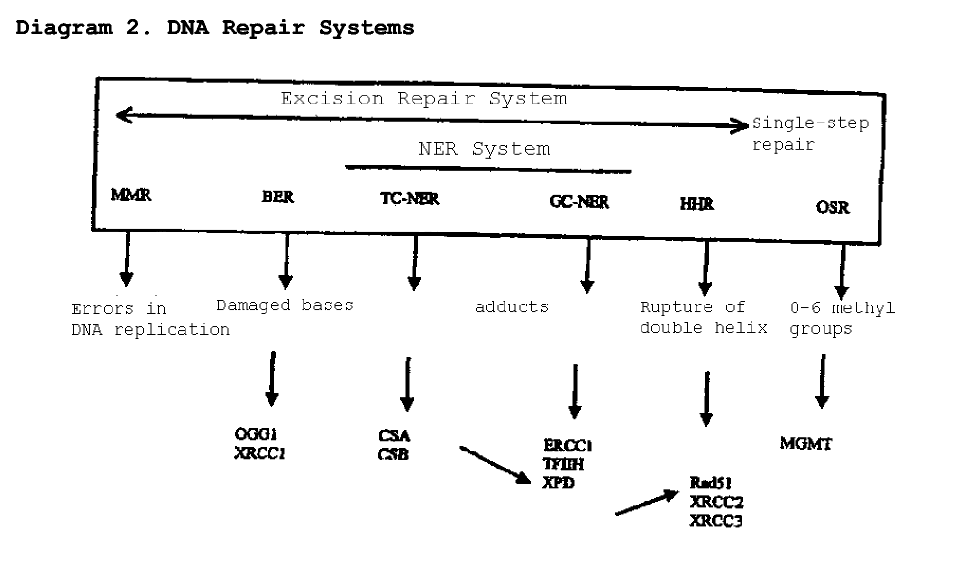

### Detailed Description of the Invention

### Classification of the Lys751Gln and Asp312Asn polymorphisms of the human XPD/ERCC2 gene.

### 1.- Gene information of the ERCC2/XPD locus

Information of the sequence of DNA, RNA and protein corresponding to this gene is detailed on the web page www.ncbi.nlm.nih.gov/locuslink/refseq.html, with Locus ID number 2068, and which is summarized below:
**ERCC2/XPD-** excision repair cross-complementing rodent repair deficiency complementation group 2 (xeroderma pigmentosum D)

### NCBI Reference Sequences (RefSeq):

mRNA : NM_000400
Protein: NP_000391
GenBank Source: X52221, X52222
mRNA : NM_000400
Protein: NP_000391

### GenBank Nucleotide Sequences:

Nucleotide: L47234 (type g), BC008346 (type m) X52221 (type m), X52222 (type m)

### Other Links:

OMIM: 126340
UniGene: Hs 99987

### 2.- Biological samples for obtaining DNA

The DNA used for the classification of the two Lys751Gln and Asp312Asn polymorphisms has been obtained from nucleated cells from peripheral blood.

It is worth pointing out that to obtain the DNA and the subsequent classification, any other nucleated cell type of the human organism can be used.

### 3.- Blood extraction

Peripheral blood is collected in vacutainer-type tubes containing K₃/EDTA (Becton Dickinson Systems; reference number 36752 or 368457). Then it is centrifuged for 15 minutes at 2,500 rpm at room temperature, and the plasma fraction is discarded. Two volumes of erythrocyte lysing solution (155 mM NH₄Cl, 0.1 mM EDTA, 10 mM Hepes, pH=7.4) are added to the cell fraction and is incubated at room temperature for 30 minutes on a rotating platform. Then the sample is centrifuged for 10 minutes at 3,000 rpm at room temperature, the supernatant is discarded and the cellular precipitate obtained is re-suspended in 1 ml of erythrocyte lysing solution. The 10-minute, 3,000 rpm centrifugation at room temperature is repeated and the supernatant is discarded. The obtained precipitate corresponds to the erythrocyte-free cell fraction.

### 4.- DNA extraction

The DNA is extracted from the peripheral blood nucleated cells and purified by means of the commercial kit QIAmp® DNA blood Mini-kit (Qiagen; reference 51104 or 51106) following the manufacturer instructions.

### 5.- Classification of the Lys751Gln and Asp312Asn polymorphisms

The following PCR conditions were used to classify the Asp312Asn polymorphism of exon 10 (final reaction volume of 25 µl) : 900 nM of primer SEQ ID NO. 1: ACGCCCACCTGGCCA, 900 nM of primer SEQ ID NO 2: GGCGGGAAAGGGACTGG, 300 nM of TaqMan MGB™ VIC probe SEQ ID NO 3: CCGTGCTGCCCGACGAAGT TAMRA, 300 nM of TaqMan MGB™ 6-FAM probe SEQ ID NO 4: CCCGTGCTGCCCAACGAAG TAMRA, 12.5 µl of TaqMan Universal PCR Master Mix (Applied Biosystems; reference 4304437) and 200 ng of DNA. The PCR cycles (50°C for 2 minutes, 95°C for 10 minutes, [92°C for 15 seconds, 60°C for 1 minute] for 40 cycles) and the polymorphism analysis were carried out in an ABI Prism 7000 Sequence Detection System equipment (Applied Biosystems) using the Allelic Discrimination program (Applied Biosystems).

The following PCR conditions were used to classify the Lys751Gln polymorphism of exon 23 (final reaction volume of 25 µl) : 900 nM of primer SEQ ID NO. 5: GCCTGGAGCAGCTAGAATCAGA, 900 nM of primer SEQ ID NO 6: CACTCAGAGCTGCTGAGCAATC, 300 nM of TaqMan MGB™ VIC probe SEQ ID NO 7: TATCCTCTGCAGCGTC TAMRA, 300 nM of TaqMan MGB™ 6-FAM probe SEQ ID NO 8: CTATCCTCTTCAGCGTC TAMRA, 12.5 µl of TaqMan Universal PCR Master Mix (Applied Biosystems; reference 4304437) and 200 ng of DNA. The PCR cycles (50°C for 2 minutes, 95°C for 10 minutes, [92°C for 15 seconds, 60°C for 1 minute] for 40 cycles) and the polymorphism analysis were carried out in an ABI Prism 7000 Sequence Detection System equipment (Applied Biosystems) using the Allelic Discrimination program (Applied Biosystems).

In both cases, the design of the primers and probes was carried out by means of the PrimerExpress™ computer program (Applied Biosystems), following the supplier instructions and using the previously described reference DNA sequence. The specificity of the primers and of the probes was previously tested by means of the BLAST computer program (www.ncbi.nlm.nih.gov/blast) . In all cases, both the primers and the probes showed unique specificity on each one of the two regions to be studied of the ERCC2/XPD gene.

### 6.- Validation of the analysis by means of automatic DNA sequencing

As validation of the obtained results, the DNA fragments corresponding to the Lys751Gln and Asp312Asn polymorphisms in 100 samples of DNA which had previously been analyzed (see previous sections) were sequenced.

In the first place, the exon 10 fragment of the XPD/ERCC2 gene where the Asp312Asn polymorphism is mapped was amplified by means of the PCR technique. The PCR reaction conditions were the following (final volume of 50 µl) : 0.25 µM of primer SEQ ID NO: 1, 0.25 µM of primer SEQ ID NO: 2, 5 µl of PCR buffer (67 mM Tris-HCl, 16.6 mM (NH₄) ₂SO₄, 0.1% Tween 20) (Ecogen; reference ETAQ-500), 1 mM MgCl₂ (Ecogen; reference ETAQ-500), 0.12 mM of PCR Nucleotide Mix (Roche; reference 1581295), 1 unit of EcoTaq DNA Polymerase (Ecogen; reference ETAQ-500) and 200 ng of DNA. The PCR cycles used were: 95°C for 5 minutes, [94°C for 30 seconds, 60°C for 45 seconds, 72°C for 1 minute] for 35 cycles, 74°C for 7 minutes.

In the second place, the exon 23 fragment of the XPD/ERCC2 gene where the Lys751Gln polymorphism is mapped was amplified by means of the PCR technique. The PCR reaction conditions were the following (final volume of 50 µl) : 0.25 µM of primer SEQ ID NO: 6, 0.25 µM of primer SEQ ID NO: 7, 5 µl of PCR buffer (67 mM Tris-HCl, 16.6 mM (NH₄)₂SO₄, 0.1% Tween 20) (Ecogen; reference ETAQ-500), 1 mM MgCl₂ (Ecogen; reference ETAQ-500), 0.12 mM PCR Nucleotide Mix (Roche; reference 1581295), 1 unit of EcoTaq DNA Polymerase (Ecogen; reference ETAQ-500) and 200 ng of DNA. The PCR cycles used were: 95°C for 5 minutes, [94°C for 30 seconds, 64°C for 45 seconds, 72°C for 1 minute] for 35 cycles, 74°C for 7 minutes.

The integrity of the PCR products was analyzed after electrophoresis in a 1.5%-TBE agarose gel and subsequent staining with 1% ethidium bromide in a UV transilluminator.

The obtained PCR products were used for the sequencing reaction as detailed as follows: in the first place, the products were purified by means of adding 4 µl of ExoSap-IT (USB; reference 7820) to 10 µl of the corresponding PCR product and was sequentially incubated at 37°C for 45 minutes and at 80°C for 15 minutes. Four µl of BigDye Terminator solution, version 3.0 (Applied Biosystems; reference 439024801024) and 3.2 pmoles of the corresponding primer (in this case, the same primers as those used in the PCR amplification, both forward and reverse, were used in separate reactions) were added to 500-600 ng of purified PCR product. The PCR cycles for this sequencing reaction were: 94°C for 5 minutes, [96°C for 10 seconds, 50°C for 5 seconds, 60°C for 4 minutes] for 32 cycles.

Once the sequencing reaction concluded, the products precipitated by means of adding 62.5 µl of 96% ethanol, 3 µl of 3 M sodium acetate buffer pH=4.6 and 24.5 µl of double-distilled water. After an incubation of 30 minutes at room temperature, they were centrifuged for 30 minutes at 14,000 rpm at room temperature, the supernatant is discarded and a washing is carried out with 250 µl of 70% ethanol. Then the samples were centrifuged for 5 minutes at 14,000 rpm at room temperature, the ethanol remains are discarded (leaving the precipitates to completely dry), and 15 µl of TSR loading buffer (Applied Biosystems; reference 401674) are added. They are finally incubated at 95°C for 3 minutes prior to their injection in the ABI Prism 310 Sequence Detection System automatic capillary equipment (Applied Biosystems). The automatic sequencing results were analyzed with the Sequencing Analysis 4.3.1 program (Applied Biosystems).

In all the analyzed cases, the two polymorphisms of each one of the samples were sequenced both with the forward primer and with the reverse primer, the results in all cases being coincident between one another and also with the results obtained by quantitative real time PCR analysis.

### Results

Three studies in metastatic lung cancer patients commenced in August of 2001 for the purpose of confirming that the allelic variants of XPD could affect survival after treatment with chemotherapy in metastatic lung cancer. These three different studies are: the first one with gemcitabine and cisplatin, the second one with vinorelbine and cisplatin and the third one with docetaxel and cisplatin. One-hundred patients with locally advanced lung cancer who underwent neoadjuvant chemotherapy and then surgery were also retrospectively analyzed. About 150 patients in initial stages who received treatment either with surgery alone or with pre-operative or post-operative chemotherapy, and whose summary is also included in the appendix, were also analyzed.

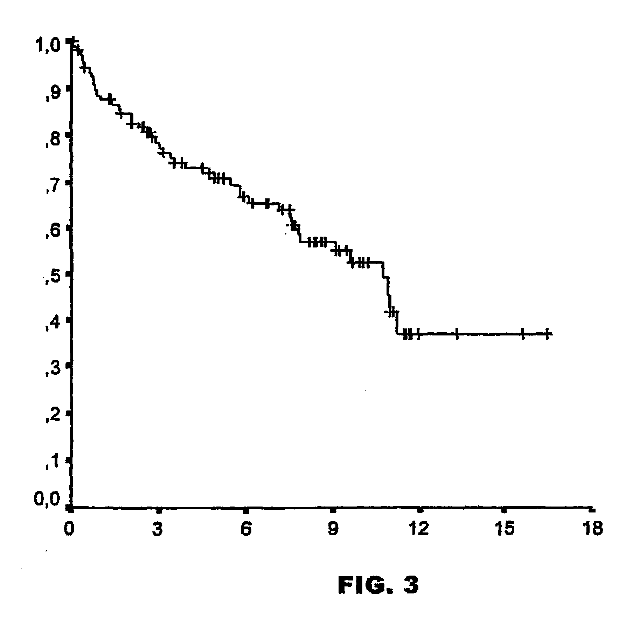

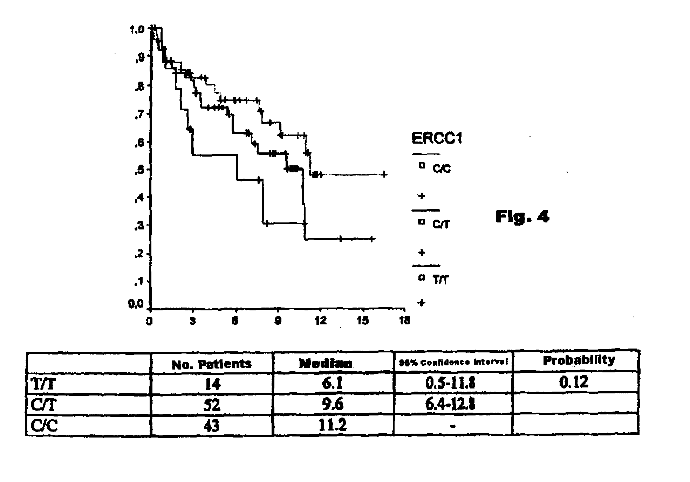

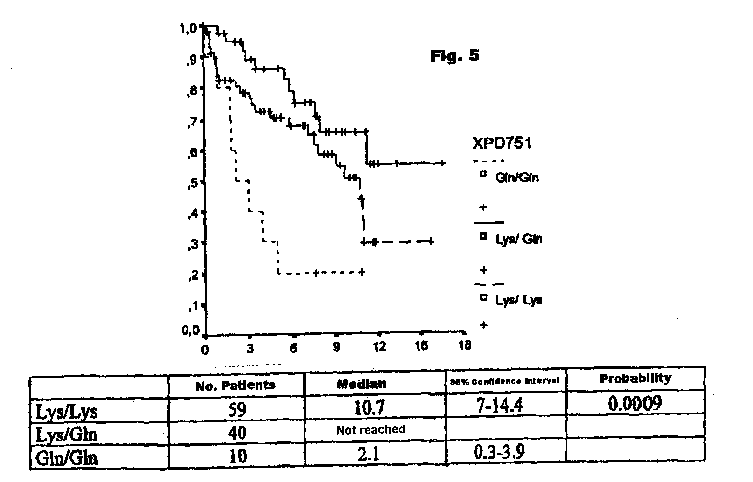

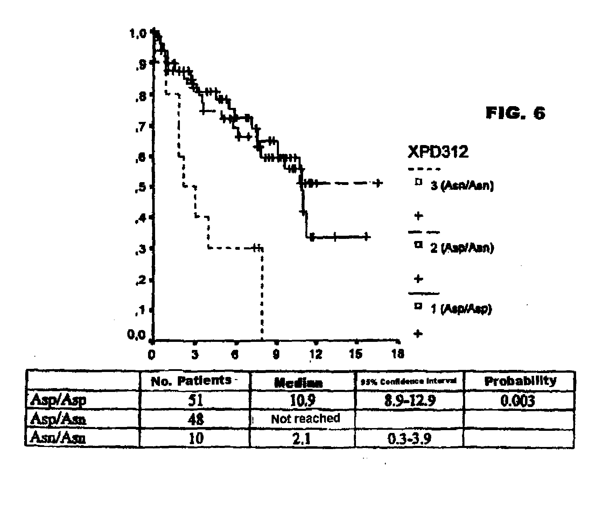

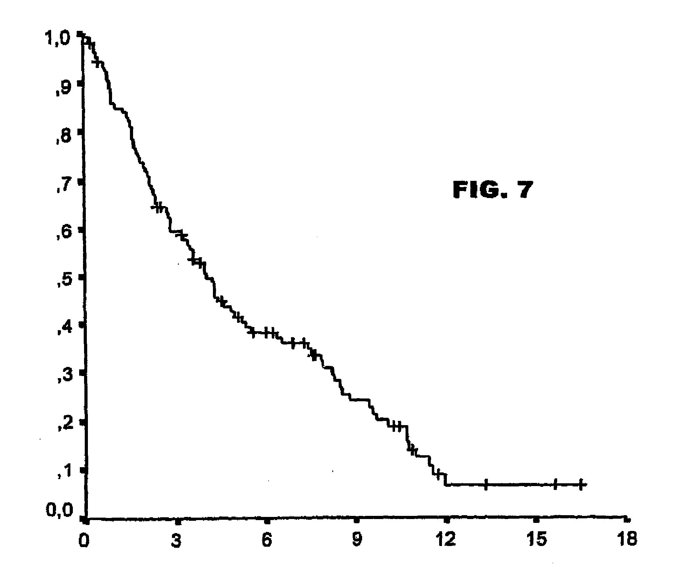

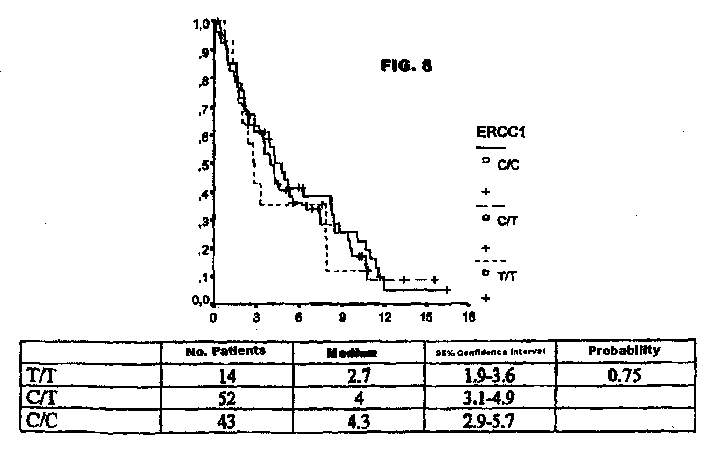

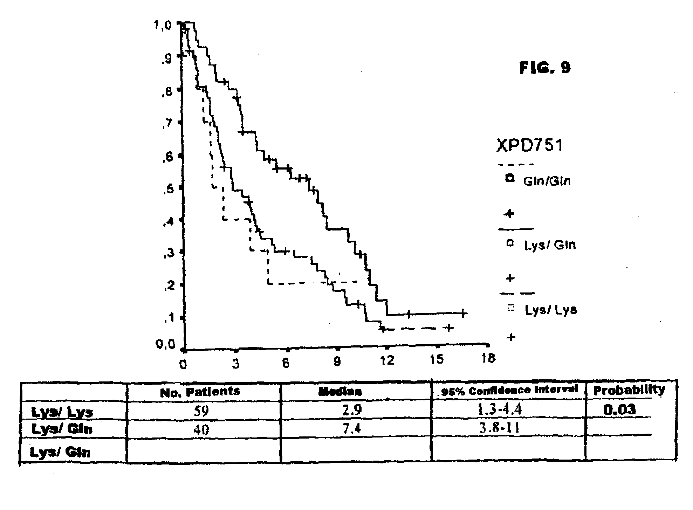

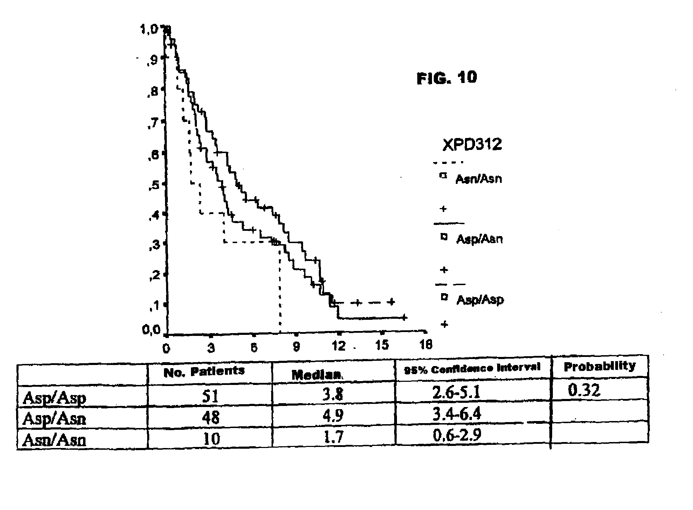

The most significant data to date are those obtained from the study of patients with stage IV lung cancer who received treatment with gemcitabine and cisplatin. Between August of 2001 and July of 2002, 250 patients were included, out of which patients final data on 109 of them is available. Attached Table 1 describes the clinical characteristics of these patients which are the normal characteristics in relation to age, general condition, histology, metastases. Table II shows the frequencies of the different polymorphisms. The polymorphism of the ERCC1 gene at position 118 was also analyzed. It can be seen that the frequencies of the XPD polymorphisms at exons 23 and 10 show that the normal homozygote genotypes constitute 50%, whereas the heterozygote variants are 40% (Table II). In the following figures, the overall survival of the 109 patients with a median survival time of 10.7 months in a range of 8.9-12.5 (Figure 3) is presented in a serial manner. The differences according to the polymorphism of the ERCC1 gene are not significant (Figure 4). However, when survival time is analyzed on the basis of the XPD polymorphism at codon 751, it is shown that the median survival time for 59 patients with the Lys/Lys genotype is 10.7 months, whereas it is much higher and the median has not yet been reached in 40 Lys/Gln heterozygote patients (Figure 5). It has also been discovered that a minority group of patients (10) are homozygotes for the Gln/Gln variant, the median survival time is 2.1 (p=0.0009) (Figure 5). The same significant differences are observed for codon 312, see the corresponding figure (p=0.003) (Figure 6). In the same manner, when the time to progression is analyzed, overall, the median time to progression is 4 months in a range of 3.2-4.8 (Figure 7). There are no differences according to the ERCC1 genotype (Figure 8). However, on the basis of the genotype, large differences are observed at codon 751, such that in the 59 patients who are Lys/Lys, the median is 2.9 months, whereas in the 40 Lys/Gln patients, the median increases to 7.4 months. The difference is very significant (p=0.03) (Figure 9). The time to progression of the XPD polymorphism at codon 312 is also shown, where the difference in survival time is not significant (Figure 10). The conclusions of this study are revealing as they differentiate two patient subgroups, some patients with a response and survival time far exceeding the overall response and survival time in which gemcitabine and cisplatin obtain great results, whereas in the other group of patients, said treatment would clearly be contraindicated in light of such meager results, far below the normally accepted median survival times.

**Table I.**

| **Clinical Characteristics of Patients Treated with Gem/Cis** | |
|---|---|
| No. of Patients | 109 |
| Age, years | 61 |
| (Medicine, range) | 35-82 |

| Clinical condition (Performance Status) | |
|---|---|
| 0-1 | 89(81.7) |
| 2 | 20(18.3) |

| Histology | |
|---|---|
| Adenocarcinoma | 52(47.7) |
| SCC | 37(33.9) |
| LCUC | 5 (4.6) |
| Others | 15(13.8) |

| Phase | |
|---|---|
| IIIb | 29(26.6) |
| IV | 80(73.4) |
| Pleural Effusion | 19(17.4) |
| Surgery | 10(9.2) |
| Radiotherapy | 11(10.1) |

| Metastasis | |
|---|---|
| Liver | 9(8.3) |
| Lung | 43(39.4) |
| Bone | 21(19.3) |
| CNS | 16(14.7) |
| Adrenal | 18(16.5) |
| Foot | 7(6.4) |
| Lymphatic nodes | 23(21.1) |
| Others | 13(11.9) |

**Table II.**

| **ERCC1 and XPD Genotypes and Response** | |
|---|---|
| Response | |
| Complete response | 5(5.3) |
| Partial response | 29(30.9) |
| Complete response + Partial response | 34(36.2) |
| Stable disease | 14(14.9) |
| Progressive disease | 46(48.9) |
| Cannot be evaluated | 15 |

| ERCC1 | |
|---|---|
| T/T | 14(12.8) |
| C/T | 52(47.7) |
| C/C | 43(39.4) |

| XPD23 | |
|---|---|
| Lys/Lys | 59(54.1) |
| Lys/Gln | 40(36.7) |
| Gln/Gln | 10(9.2) |

| XPD10 | |
|---|---|
| Asp/Asp | 51(46.8) |
| Asp/Asn | 48(44) |
| Asn/Asn | 10(9.2) |

In a second stage IV lung cancer study, which also commenced in August of 2001, about 100 patients treated with cisplatin and vinorelbine were analyzed, and of which patients preliminary results are available. The effect of vinorelbine according to the XPD genotype shows that when Lys/Lys patients with a poor prognosis are treated with gemcitabine and cisplatin, in this case, when vinorelbine is used, the opposite occurs and a time to progression of 10 months is obtained in the Lys/Lys patient group when they are treated in the study with gemcitabine and cisplatin, said median time to progression is only 2.9 months. See the corresponding Graphs 11 and 12.

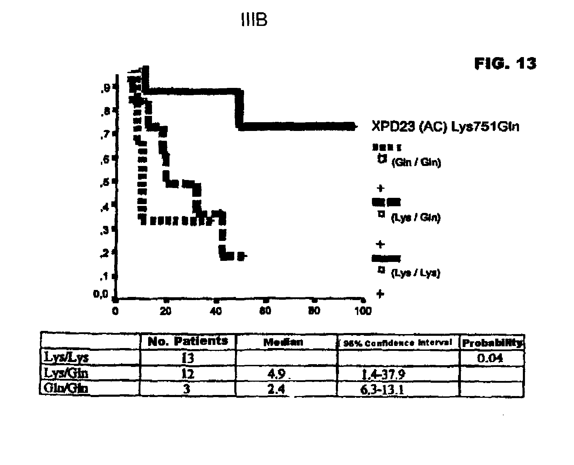

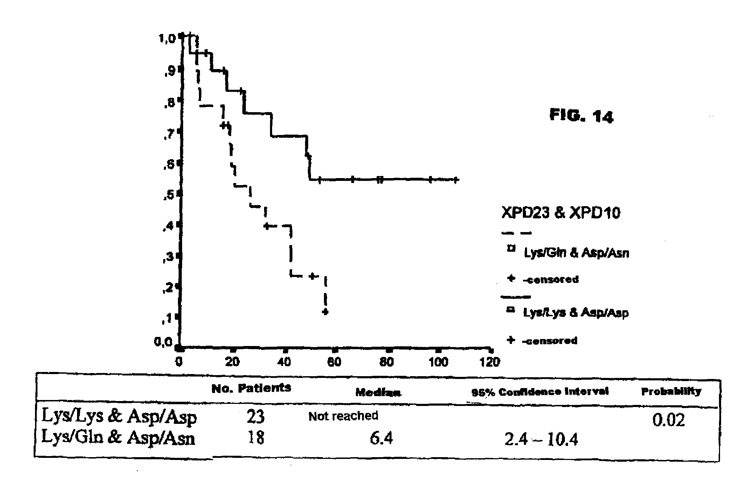

Finally, the results of the XPD polymorphism in locally advanced, stage III lung cancer patients, where once again survival time varies according to the genotype, are also shown. By adding docetaxel to the gemcitabine and cisplatin combination, the time to progression is significantly greater in Lys/Lys plus Asp/Asp or Lys/Lys homozygote patients. See corresponding Figures 13 and 14.

### Clinical Application

These results unequivocally signal the individual pharmacogenetic prediction of lung cancer for the first time. First, the Lys751Gln XPD genotype predicts an effect and a survival time substantially greater than normal when treated with gemcitabine and cisplatin. Secondly, said combination is clearly contraindicated in the other Lys751Lys and Gln751Gln genotypes. Clinical results also show that Lys751Lys patients respond very favorably to the combination of vinorelbine and cisplatin or docetaxel and cisplatin. Finally and in the third place, it is identified that a minority patient group with the Gln751Gln genotype have a very poor survival time with any combination of chemotherapy with cisplatin, and therefore they should be treated with combinations without cisplatin.

The XPD polymorphism genetic test is absolutely necessary for the appropriate selection of drugs prior to administering chemotherapy in cancer patients, and very particularly in lung cancer patients.

### Description of the Figures

Figure 1: XPD 312 polymorphism with G → A substitution causing an amino acid change of Asp → Asn at codon 312.
Figure 2: XPD 751 polymorphism with A → C substitution causing an amino acid change of Lys → Gln at codon 751.
Figure 3: Abscissa: months; Ordinate: Probability. Overall survival time with Gem/Cis.
Figure 4: Abscissa: months; Ordinate: Probability. Survival time according to ERCC1 genotype.
Figure 5: Abscissa: months; Ordinate: Probability. Survival time according to XPD 751.
Figure 6: Abscissa: months; Ordinate: Probability. Survival time according to XPD 312.
Figure 7: Abscissa: months; Ordinate: Probability. Time to progression.
Figure 8: Abscissa: months; Ordinate: Probability. Progression according to ERCC1 genotype.
Figure 9: Abscissa: months; Ordinate: Probability. Progression according to XPD 751 genotype.
Figure 10: Abscissa: months; Ordinate: Probability. Progression according to XPD 312 genotype.

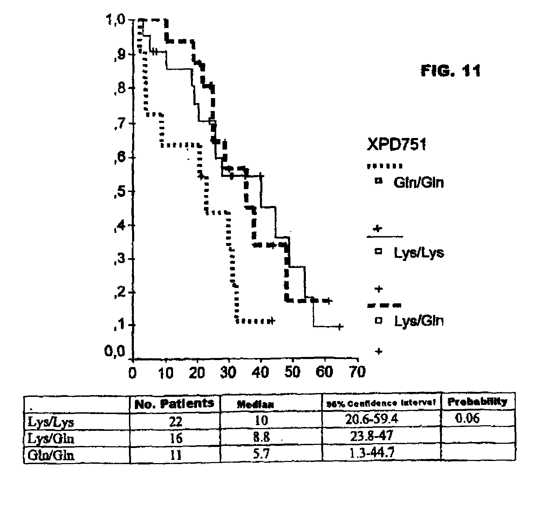

Figure 11: Abscissa: months; Ordinate: Probability. Progression according to XPD 751 genotype for vinorelbine/cisplatin.

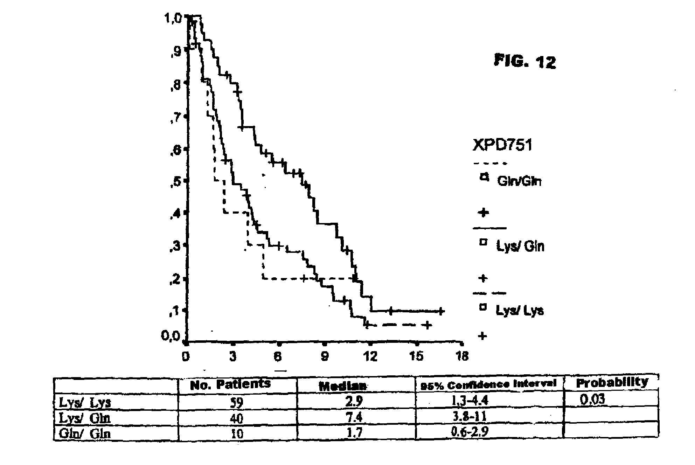

Figure 12: Abscissa: months; Ordinate: Probability. Progression according to XPD 751 genotype for gemcitabine/cisplatin.
Figure 13: Abscissa: weeks; Ordinate: Probability. Progression according to XPD 751 genotype for Gem/Cis/Docetaxel.
Figure 14: Abscissa: weeks; Ordinate: Probability. Progression according to XPD 751 and 312 genotypes.

## Claims

1. An assay device for detecting the genetic predisposition to respond to treatment of antitumor drugs useful for lung cancer based on the detection of polymorphisms and/or loss of heterozygosity in the ERCC2/XPD repair gene, the locus of which is defined by GenBank sequences X52221 and X52222, **characterized by** comprising at least one of the oligonucleotide probes selected among SEQ ID NO: 1 and SEQ ID NO: 2; or SEQ ID NO: 5 and SEQ ID NO: 6.

2. An assay device, **characterized in that** the probes are used as human DNA sample mapping primers in polymerase chain reaction (PCR) reaction technique.

3. An assay device, **characterized in that** the probes are used as human DNA sample mapping primers in automatic sequencing technique.

4. An assay device according to any of claims 1 to 3, **characterized by** detecting Lys751Gln or Asp312Asn polymorphisms, respectively, for each pair of primers.

5. An assay device according to any of claims 1 to 4, **characterized in that** the antitumor drug is a combination of cisplatin with a second antitumor compound selected among gemcitabine, vinorelbine or docetaxel.

6. The oligonucleotide primers for detecting the genetic predisposition to the response to antitumor drugs, **characterized by** the sequences represented by SEQ ID NO: 1, SEQ ID NO: 2, SEQ ID NO: 5 and SEQ ID NO: 6.

7. The oligonucleotides according to claim 6, **characterized in that** the antitumor drug is a combination of cisplatin with a second antitumor compound selected among gemcitabine, vinorelbine or docetaxel.

8. The use as primers for detecting the genetic predisposition to the response to antitumor drugs of the oligonucleotides represented by SEQ ID NO: 3, SEQ ID NO: 4, SEQ ID NO: 7 or SEQ ID NO: 8.

9. The use according to claim 3, **characterized in that** the antitumor drug is a combination of cisplatin with a second antitumor compound selected among gemcitabine, vinorelbine or docetaxel.
